# FASCICULE DE BREVET EUROPEEN

(11) **EP 0 943 320 B1**
(45) Date de publication et mention de la délivrance du brevet: **14.11.2001**
(21) Numéro de dépôt: 99400505.6
(22) Date de dépôt: 02.03.1999
(51) Int. Cl.: A61K 7/13

(54) **Procédé de teinture d'oxidation et composition de teinture d'oxidation pour fibres kératiniques comprenant un polymère amphiphile cationique**
Verfahren zum oxidativen Färben und Zusammensetzung zum oxidativen Färben von keratinfasern, die ein kationisches amphiphiles Polymer enthält
Process for oxidative dyeing and composition for oxidative dyeing of keratinic fibres comprising a cationic amphiphilic polymer

(30) Priorité: 06.03.1998 FR 9802775
(43) Date de publication de la demande: 22.09.1999
(73) Titulaire: L'OREAL, 75008 Paris (FR)
(72) Inventeur: Laurent, Florence, 92600 Asnières (FR); De La Mettrie, Roland, 78110 Le Vésinet (FR)
(74) Mandataire: Casalonga, Axel

(56) Documents cités:
- EP-A- 0 046 543
- EP-A- 0 763 355
- FR-A- 2 312 233
- FR-A- 2 331 325
- FR-A- 2 695 033
- GB-A- 2 096 180

## Description

La présente invention concerne un procédé de teinture d'oxydation des fibres kératiniques, et en particulier des fibres kératiniques humaines telles que les cheveux, avec des compositions comprenant, dans un milieu approprié pour la teinture, au moins un précurseur de colorant d'oxydation, éventuellement, un ou plusieurs coupleurs, au moins un agent oxydant ainsi qu'au moins un polymère amphiphile cationique.

Il est connu de teindre les fibres kératiniques, et en particulier les cheveux, avec des compositions de teinture contenant des précurseurs de colorants d'oxydation, généralement appelés "bases d'oxydation", en particulier des *ortho-* ou *para*-phénylènediamines, des *ortho-* ou *para*-aminophénols et des bases hétérocycliques.

Les précurseurs de colorants d'oxydation sont des composés initialement peu ou pas colorés qui développent leur pouvoir tinctorial au sein du cheveu en présence d'un agent oxydant. La formation des composés colorés résulte, soit d'une condensation des "bases d'oxydation" sur elles-mêmes, soit d'une condensation des "bases d'oxydation" sur des composés modificateurs de coloration, ou "coupleurs", qui sont généralement présents dans les compositions tinctoriales utilisées en teinture d'oxydation et qui sont représentés plus particulièrement par des *méta*-phénylènediamines, des *méta*-aminophénols et des *méta*-diphénols et certains composés hétérocycliques.

La variété des molécules mises enjeu, qui sont constituées d'une part par "les bases d'oxydation" et, d'autre part, par les "coupleurs", permet l'obtention d'une palette riche en coloris.

Lors de l'application du produit de coloration sur les cheveux, il est nécessaire de maintenir celui-ci à l'endroit de l'application et d'éviter qu'il ne coule sur le visage ou en dehors des zones que l'on souhaite teindre. Pour cela, on a eu recours jusqu'ici à l'emploi d'épaississants traditionnels tels que le poly(acide acrylique) réticulé, les hydroxyéthylcelluloses, des cires ou encore certains agents tensioactifs non ioniques qui, convenablement choisis, donnent lieu à en effet épaississant, voire gélifiant, des milieux aqueux.

Cependant, la demanderesse a constaté que les ingrédients du type épaississants traditionnels, tensioactifs et solvants, freinent généralement la montée du colorant sur les fibres, ce qui se traduit par des nuances moins lumineuses. Pour obtenir une chromaticité équivalente, il est alors nécessaire d'utiliser des quantités plus importantes de colorants ainsi que davantage de solvant et/ou d'agents tensioactifs pour dissoudre ceux-ci.

La demanderesse a également constaté que les compositions contenant le ou les précurseurs de colorants d'oxydation et, éventuellement, le ou les coupleurs, et épaissies par des épaississants traditionnels, perdaient une partie de leur caractère gélifié lorsqu'on les mélangeait avec la composition contenant l'agent d'oxydation.

Après d'importantes recherches effectuées dans ce domaine, la demanderesse vient de découvrir que l'introduction d'une quantité efficace d'un polymère associatif cationique particulier , en tant qu'épaississant,
(i) soit dans la composition contenant le ou les précurseurs de colorants d'oxydation et, éventuellement, le ou les coupleurs (Composition A),
(ii) soit dans la composition oxydante (Composition B),
(iii) soit dans les deux compositions (A et B) à la fois, permettait d'obtenir des compositions de teinture d'oxydation qui, même après mélange avec l'oxydant, ne coulent pas et restent par conséquent mieux localisées au point d'application. Ces compositions donnent par ailleurs naissance à des nuances plus chromatiques (plus lumineuses) et plus puissantes que ne le font des compositions équivalentes contenant des systèmes épaississants habituels.

Les colorations obtenues présentent par ailleurs une bonne résistance à la transpiration.

Au sens de la présente invention, la chromaticité (luminosité) est définie par la valeur C* dans le système de notation colorimétrique L*, a*, b* de la Commission Internationale de l'Éclairage (C. I. E). Cette valeur est égale à la racine carrée de la somme a² + b² (+a est rouge, -a est vert, +b est jaune, -b est bleu). Une nuance est d'autant plus lumineuse que la valeur de C* est grande. Dans ce système de notation, L* définit la puissance de la nuance. La nuance est d'autant plus puissante que la valeur de L* est faible (0 = noir, 100 = blanc).

Grâce à la présente invention, il est en outre possible de réduire de manière avantageuse, voire de supprimer, l'utilisation d'agents tensioactifs.

L'invention permet également de diminuer la quantité de matières actives colorantes utilisées dans les compositions de teinture par rapport aux techniques classiques et connues de l'art antérieur.

Selon la présente invention, on entend par "polymères associatifs" des polymères hydrosolubles capables, dans un milieu aqueux, de s'*associer* réversiblement entre eux ou avec d'autres molécules. La structure chimique de ces polymères, appelés également "polymères amphiphiles", est caractérisée par la présence de zones hydrophiles assurant la solubilité dans l'eau, et de zones hydrophobes par lesquelles les polymères, dans un milieu aqueux, s'assemblent entre eux ou avec les parties hydrophobes d'autres molécules.

La présente invention a ainsi pour objet un procédé de teinture des fibres kératiniques et en particulier des fibres kératiniques humaines telles que les cheveux, consistant à appliquer sur les fibres une composition de teinture d'oxydation (A) contenant, dans un milieu approprié pour la teinture, au moins un précurseur de colorant d'oxydation et éventuellement un où plusieurs coupleurs, et à révéler la couleur en milieu alcalin, neutre ou acide à l'aide d'une composition oxydante (B) contenant un agent oxydant, l'une au moins des compositions (A) et (B) contenant, en outre, une quantité efficace d'au moins un polymère amphiphile cationique choisi parmi :
- les celluloses quaternisées modifiées par des groupements comportant au moins une chaîne grasse, choisis parmi les groupes alkyle, arylalkyle, alkylaryle comportant au moins 8 atomes de carbone, ou des mélanges de ceux-ci, et
- les hydroxyéthylcelluloses quaternisées modifiées par des groupements comportant au moins une chaîne grasse, choisis parmi les groupes alkyle, arylalkyle, alkylaryle comportant au moins 8 atomes de carbone, ou des mélanges de ceux-ci,

les compositions (A) et (B) étant mélangées immédiatement avant l'emploi ou appliquées l'une après l'autre sur les fibres kératiniques.

L'invention a également pour objet une composition de teinture d'oxydation pour fibres kératiniques, en particulier pour fibres kératiniques humaines, comprenant, dans un milieu approprié pour la teinture, au moins un précurseur de colorant d'oxydation, le cas échéant, un ou plusieurs coupleurs et au moins un polymère amphiphile cationique choisi parmi :
- les celluloses quaternisées modifiées par des groupements comportant au moins une chaîne grasse, choisis parmi les groupes alkyle, arylalkyle, alkylaryle comportant au moins 8 atomes de carbone, ou des mélanges de ceux-ci, et
- les hydroxyéthylcelluloses quaternisées modifiées par des groupements comportant au moins une chaîne grasse, choisis parmi les groupes alkyle, arylalkyle, alkylaryle comportant au moins 8 atomes de carbone, ou des mélanges de ceux-ci.

Un troisième objet de l'invention est une composition oxydante servant à révéler la couleur d'une composition de teinture d'oxydation et comprenant au moins un agent oxydant et au moins un polymère amphiphile cationique tel que défini ci-dessus.

Un autre objet de la présente invention est une composition prête à l'emploi pour la teinture des fibres kératiniques, contenant au moins un précurseur de colorant d'oxydation, éventuellement un ou plusieurs coupleurs, au moins un polymère amphiphile cationique tel que défini ci-dessus et au moins un agent oxydant.

L'invention a également pour objet des dispositifs de teinture à plusieurs compartiments, ou "kits" de teinture comportant au moins deux compartiments, dont l'un contient une composition (A) comprenant, dans un milieu approprié pour la teinture, au moins un précurseur de colorant d'oxydation et éventuellement un ou plusieurs coupleurs, et un autre contient une composition oxydante (B) comprenant au moins un agent oxydant, l'une au moins des compositions (A) et (B) comprenant, en outre, une quantité efficace d'au moins un polymère amphiphile cationique tel que défini ci-dessus.

Les radicaux alkyle portés par les celluloses ou hydroxyéthylcelluloses quaternisées ci-dessus comportent de préférence de 8 à 30 atomes de carbone. Les radicaux aryle désignent de préférence les groupements phényle, benzyle, naphtyle ou anthryle.

On peut indiquer comme exemples d'alkylhydroxyéthylcelluloses quaternisées à chaînes grasses en C₈₋₃₀, les produits QUATRISOFTLM 200, QUATRISOFT LM-X 529-18-A, QUATRISOFT LM-X 529-18B (alkyle en C₁₂) et QUATRISOFT LM-X 529-8 (alkyle en C₁₈) commercialisés par la société AMERCHOL et les produits CRODACEL QM, CRODACEL QL (alkyle en C₁₂) et CRODACEL QS (alkyle en C₁₈) commercialisés par la société CRODA.

Les polymères amphiphiles cationiques utilisés dans les compositions de la présente invention sont présents de préférence à raison de 0,05 à 10 % en poids, en particulier à raison de 0,1 à 5 % en poids par rapport au poids de la composition de teinture d'oxydation (A) ou de la composition oxydante (B).

Les précurseurs de colorants d'oxydation utilisables dans le cadre de la présente invention sont choisis parmi ceux classiquement connus en teinture d'oxydation. On peut citer notamment :
- les *para*-phénylènediamines de formule (I) suivante et les sels d'addition d'un acide de ces composés 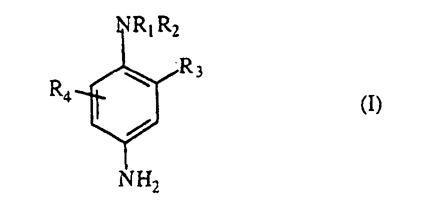 dans laquelle
   R₁ représente un atome d'hydrogène, un radical alkyle en C₁₋₄, monohydroxyalkyle en C₁₋₄, polyhydroxyalkyle en C₂₋₄ ou 4'-aminophényle,
   R₂ représente un atome d'hydrogène, un radical alkyle en C₁₋₄, monohydroxyalkyle en C₁₋₄ ou polyhydroxyalkyle en C₂₋₄,
   R₃ représente un atome d'hydrogène, un atome d'halogène tel qu'un atome de chlore, un radical alkyle en C₁₋₄, sulfo, carboxy, monohydroxyalkyle en C₁₋₄ ou hydroxyalcoxy en C₁₋₄,
   R₄ représente un atome d'hydrogène ou un radical alkyle en C₁₋₄.

Parmi les *para*-phénylènediamines de formule (I) ci-dessus, on peut citer en particulier la *para*-phénylènediamine, la *para*-toluylènediamine, la 2-chloro-*para*-phénylènediamine, la 2,3-diméthyl-*para*-phénylènediamine, la 2,6-diméthyl-*para*-phénylènediamine, la 2,6-diéthyl-*para*-phénylènediamine, la 2,5-diméthyl-*para*-phénylènediamine, la N,N-diméthyl-*para*-phénylènediamine, la N,N-diéthyl-*para*-phénylènediamine, la N,N-dipropyl*-para*-phénylènediamine, la 4-amino-N,N-diéthyl-3-méthylaniline, la N,N-bis(β-hydroxyéthyl)*-para*-phénylènediamine, la 4-amino-N,N-bis(β-hydroxyéthyl)-3-méthylaniline, la 4-amino-3-chloro-N,N-bis(β-hydroxyéthyl)-aniline, la 2-β-hydroxyéthyl*-para*-phénylènediamine, la 2-fluoro*-para*-phénylènediamine, la 2-isopropyl*-para*-phénylène, la N-(β-hydroxypropyl)-*para*-phénylènediamine, la 2-hydroxyméthyl*-para*-phénylènediamine, la N,N-diméthyl-3-méthyl*-para*-phénylènediamine, la N-éthyl-N-β-hydroxyéthyl)*-para*-phénylènediamine, la N-(β,γ-dihydroxypropyl)*-para*-phénylènediamine, la N-(4'-aminophényl)*-para*-phénylènediamine, la N-phényl-*para*-phénylènediamine, la 2-β-hydroxyéthyloxy-*para*-phénylènediamine et les sels d'addition d'acide de ces composés.

Parmi les *para*-phénylènediamines de formule (I) ci-dessus, on préfère tout particulièrement la *para*-phénylènediamine, la *para*-toluylènediamine, la 2-isopropyl*-para*-phénylènediamine, la 2-β-hydroxy-éthyl*-para*-phénylèndiamine, la 2-β-hydroxyéthyloxy*-para*-phénylènediamine, la 2,6-diméthyl*-para*-phénylènediamine, la 2,6-diéthyl*-para*-phénylènediamine, la 2,3-diméthyl*-para*-phénylènediamine, la N,N-bis-(β-hydroxyéthyl)-*para*-phénylènediamine, la 2-chloro*-para*-phénylènediamine et les sels d'addition d'acide de ces composés.
- les bis-phénylalkylènediamines de formule (II) : 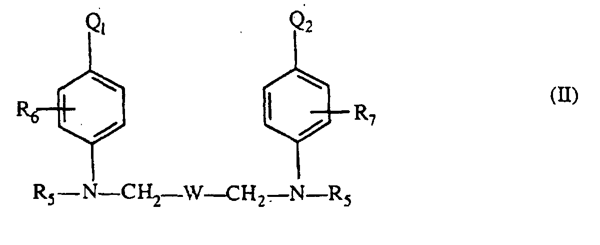 dans laquelle
   Q₁ et Q₂, identiques ou différents, représentent un radical hydroxyle ou NHR₈ dans lequel R₈ représente un atome d'hydrogène ou un radical alkyle en C₁₋₄,
   R₅ représente un atome d'hydrogène, un radical alkyle en C₁₋₄, monohydroxyalkyle en C₁₋₄, polyhydroxyalkyle en C₂₋₄ ou aminoalkyle en C₁₋₄ dont le groupe amino peut être substitué,
   R₆ et R₇, identiques ou différents, représentent un atome d'hydrogène ou d'halogène ou un radical alkyle en C₁₋₄,
   W représente un radical choisi dans le groupe formé par les radicaux suivants :

      -(CH₂)ₙ- ; -(CH₂)ₘ-O-(CH₂)ₘ ; -(CH₂)ₘ-CHOH-(CH₂)ₘ- et

      -(CH₂)ₘ-N(CH₃)-(CH₂)ₘ- ;
dans lesquels n est un nombre entier compris entre 0 et 8 inclusivement et m est un nombre entier compris entre 0 et 4 inclusivement, et les sels d'addition d'acide de tels composés.

Parmi les bis-phénylalkylènediamines de formule (II) ci-dessus, on peut citer en particulier le N,N'-bis(β-hydroxyéthyl)-N,N'-bis(4-aminophényl)-1,3-diamino-2-propanol, la N,N'-bis(β-hydroxyéthyl)-N,N'-bis(4-aminophényl)-éthylènediamine, la N,N'-bis(4-aminophényl)-tétraméthylènediamine, la N,N'-bis(β-hydroxyéthyl)-N,N'-bis(4-aminophényl)-tétraméthylènediamine, la N,N'-bis(4-méthylaminophényl)-tétraméthylènediamine, la N,N'-bis(β-hydroxyéthyl)-N,N'-bis(4-aminophényl)-tétraméthylènediamine, la N,N'-bis(éthyl)-N,N'-bis(4-amino-3-méthylphényl)-éthylènediamine, et les sels d'addition d'acide de ces composés.

On recommande en particulier parmi ces bis-phénylalkylènediamines de formule (II) le N,N'-bis(β-hydroxyéthyl)-N,N'-bis(4'-aminophényl)-1,3-diamino-2-propanol ou l'un de ses sels d'addition d'un acide.
- les *para*-aminophénols répondant à la formule (III) : 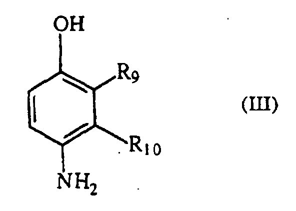 dans laquelle
   R₉ représente un atome d'hydrogène, un radical alkyle en C₁₋₄, monohydroxyalkyle en C₁₋₄, (alcoxy en C₁₋₄)-(alkyle en C₁₋₄) ou aminoalkyle en C₁₋₄, ou hydroxy(alkyle en C₁₋₄)-aminoalkyle en C₁₋₄);
   R₁₀, représente un atome d'hydrogène ou de fluor, un radical alkyle en C₁₋₄, monohydroxyalkyle en C₁₋₄, polyhydroxyalkyle en C₂₋₄, aminoalkyle en C₁₋₄, cyano(alkyle en C₁₋₄) ou (alcoxy en C₁₋₄)-(alkyle en C₁₋₄), et les sels d'addition d'acide de tels composés,
   avec la réserve qu'au moins un des radicaux R₉ ou R₁₀ représente un atome d'hydrogène.

Parmi les *para*-aminophénols de formule (III) ci-dessus, on peut citer notamment le *para*-aminophénol, le 4-amino-3-méthylphénol, le 4-amino-3-fluorophénol, le 4-amino-3-hydroxyméthyl-phénol, le 4-amino-2-méthyl-phénol, le 4-amino-2-hydroxyméthylphénol, le 4-amino-2-méthoxyméthyl-phénol, le 4-amino-2-aminométhylphénol, le 4-amino-2-(β-hydroxyéthyl-aminométhyl)-phénol, et les sels d'addition d'acide de ces composés.
- les *ortho*-aminophénols utilisables à titre de bases d'oxydation dans le cadre de la présente invention sont notamment choisis parmi le 2-aminophénol, le 2-amino-1-hydroxy-5-méthylbenzène, le 2-amino-1-hydroxy-6-méthylbenzène, le 5-acétamido-2-aminophénol et les sels d'addition d'acide de ces composés;
- les bases hétérocycliques utilisables à titre de bases d'oxydation dans le cadre de la présente invention sont notamment choisies parmi les dérivés pyridiniques, les dérivés pyrimidiniques, les dérivés pyrazoliques et les sels d'addition d'acide de ces composés.

Parmi les dérivés pyridiniques, on peut plus particulièrement citer les composés décrits par exemple dans les brevets GB-1 026 978 et GB-1 153 196, comme la 2,5-diaminopyridine et les sels d'addition d'acide de tels composés.

Parmi les dérivés pyrimidiniques, on peut citer en particulier les composés décrits par exemple dans le brevet allemand DE-2 359 399 ou les brevets japonais JP-88-169 571 et JP-91-333 495, comme la 2,4,5,6-tétraaminopyrimidine, la 4-hydroxy-2,5,6,-triaminopyrimidine et les sels d'addition d'acide de tels composés.

Parmi les dérivés pyrazoliques, on peut plus particulièrement citer les composés décrits dans les brevets DE-3 843 892, DE-4 133 957 et demandes de brevet WO-94/08969 et WO-94/08970 comme le 4,5-diamino-1-méthylpyrazole, le 3,4-diamino-pyrazole, le 4,5-diamino-1-(4'-chlorobenzyl)-pyrazole et les sels d'addition d'acide de ces composés.

Selon l'invention, le ou les précurseurs de colorants d'oxydation représentent de préférence de 0,0005 à 12 % en poids du poids total de la composition (A) et encore mieux de 0,005 à 6 % en poids environ.

Les coupleurs utilisables dans le procédé de teinture selon l'invention sont ceux classiquement utilisés dans les compositions de teinture d'oxydation, c'est-à-dire des *méta*-phénylènediamines, des *méta*-aminophénols et des *méta*-diphénols (résorcinols), les dérivés mono- ou polyhydroxylés du naphtalène, le sésamol et ses dérivés et des composés hétérocycliques tels que, par exemple, les coupleurs indoliques, les coupleurs indoliniques, les coupleurs pyridiniques et les sels d'addition d'acide de tels composés.

Ces coupleurs peuvent notamment être choisis parmi le 2-méthyl-5-amino-phénol, le 5-N-(β-hydroxyéthyl)-amino-2-méthylphénol, le 3-aminophénol, le 1,3-dihydroxybenzène, le 1,3-dihydroxy-2-méthylbenzène, le 4-chloro-1,3-dihydroxybenzène, le 1-(β-hydroxyéthoxy)-2,4-diaminobenzène, le 2-amino-4-(β-hydroxyéthylamino)-1-méthoxybenzène, le 1,3-diaminobenzène, le 1,3-bis(2,4-diaminophénoxy)-propane, le sésamol, l'α-naphtol, le 6-hydroxyindole, le 4-hydroxyindole, le 4-hydroxy-N-méthylindole, la 6-hydroxyindoline, la 2,6-dihydroxy-4-méthylpyridine, la 1-H-3-méthylpyrazol-5-one, la 1-phényl-3-méthyl-pyrazol-5-one et les sels d'addition d'acide de tels composés.

Lorsqu'ils sont présents, ces coupleurs représentent de préférence d'environ 0,0001 à 10 % en poids du poids total de la composition (A), et en particulier d'environ 0,005 à 5 % en poids.

D'une manière générale, les sels d'addition d'un acide des composés chromogènes, à savoir les bases d'oxydation et les coupleurs, sont choisis notamment parmi les chlorhydrates, les bromhydrates, les sulfates, les tartrates, les lactates et les acétates.

La composition (A) peut contenir, en plus des précurseurs de colorants d'oxydation définis ci-dessus et des éventuels coupleurs associés, des colorants directs pour enrichir les nuances en reflets. Ces colorants directs peuvent notamment être choisis parmi les colorants nitrés, azoïques ou anthraquinoniques.

La composition (A) et/ou la composition (B) peuvent en outre contenir au moins un polymère substantif cationique ou amphotère tel que ceux définis dans EP-A-0 673 641, parmi lesquels on préfère avantageusement mettre en oeuvre :
- les polymères poly(ammonium quaternaire) préparés et décrits dans le brevet français 2 270 846, constitués de motifs récurrents répondant à la formule (IV) suivante : 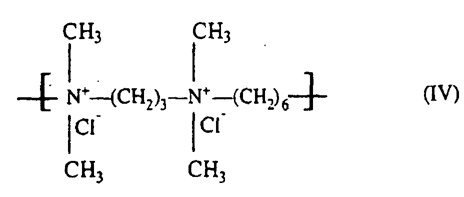 et notamment ceux dont la masse molaire moyenne en poids, déterminée par chromatographie par perméation de gel, est comprise entre 9500 et 9900 ; et
- les polymères poly(ammonium quaternaire) préparés et décrits dans le brevet français 2 270 846, constitués de motifs récurrents répondant à la formule (V) suivante : 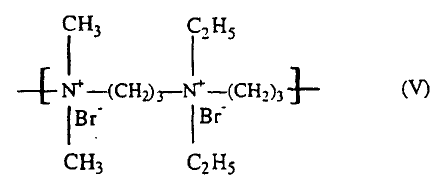 et notamment ceux dont la masse molaire moyenne en poids, déterminée par chromatographie par perméation de gel, est d'environ 1200.

Le milieu de la composition (A) approprié pour la teinture, est de préférence un milieu aqueux constitué majoritairement d'eau et contenant, éventuellement, des solvants organiques acceptables sur le plan cosmétique, parmi lesquels figurent des alcools tels que l'alcool éthylique, l'alcool isopropylique, l'alcool benzylique et l'alcool phényléthylique; des glycols ou éthers de glycols tels que les éthers monométhylique, monoéthylique et monobutylique d'éthylèneglycol, le propylèneglycol ou ses éthers tels que l'éther monométhylique de propylèneglycol; le butylèneglycol; le dipropylèneglycol ainsi que les alkyléthers de diéthylèneglycol comme par exemple l'éther monométhylique ou monobutylique de diéthylèneglycol, en des concentrations comprises entre environ 0,5 et 20 % en poids, de préférence entre environ 2 et 10 % en poids, par rapport au poids total de la composition.

La composition (A) et/ou la composition (B) peuvent encore contenir une quantité efficace d'autres agents utilisés couramment dans le domaine cosmétique. Ces adjuvants sont par exemple des agents séquestrants, des agents de conditionnement du cheveu et en particulier des silicones, des agents conservateurs, des agents opacifiants etc., et éventuellement des agents tensio-actifs anioniques, non-ioniques, amphotères ou des mélanges de ceux-ci.

Ladite composition de teinture peut contenir également des agents anti-oxydants. Ceux-ci peuvent être choisis en particulier parmi le sulfite de sodium, l'acide thioglycolique, l'acide thiolactique, le bisulfite de sodium, l'acide déshydrogénoascorbique, l'hydroquinone, la 2-méthylhydroquinone, la *tert*-butyl-hydroquinone et l'acide homogentisique. Ils sont présents, le cas échéant, à raison d'environ 0,05 à 3,0 % en poids par rapport au poids total de la composition.

Bien entendu, l'homme de métier veillera à choisir le ou les éventuels composés complémentaires mentionnés ci-avant, d'une manière telle que les propriétés avantageuses attachées intrinsèquement à la composition tinctoriale selon l'invention ne soient pas, ou pratiquement pas, altérées par la ou les adjonctions envisagées.

Dans la composition (B), l'agent oxydant est choisi de préférence parmi le peroxyde d'hydrogène, le peroxyde d'urée, les bromates ou ferricyanures de métaux alcalins, les sels de peracides tels que les perborates, les percarbonates et les persulfates. On recommande en particulier l'utilisation de peroxyde d'hydrogène.

La composition (B) est avantageusement constituée d'une solution d'eau oxygénée dont le titre varie de préférence entre environ 2,5 et 40 volumes, en particulier entre environ 5 et 20 volumes.

La valeur du pH de la composition prête à l'emploi, résultant du mélange de la composition tinctoriale (A) et de la composition oxydante (B), est généralement comprise entre 4 et 11, de préférence entre 6 et 10,5. Elle peut être ajustée au moyen d'agents acidifiants ou alcalinisants bien connus dans la technique de teinture d'oxydation des fibres kératiniques.

On peut citer parmi les agents alcalinisants, par exemple l'ammoniaque, les carbonates alcalins, les alcanolamines telles que les mono-, di- et triéthanolamines ainsi que leurs dérivés, les hydroxydes de sodium ou de potassium et les composés de formule (VI) suivante : 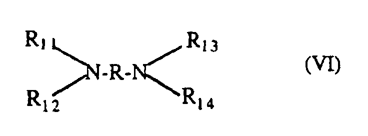 dans laquelle R est un résidu propylène éventuellement substitué par un groupement hydroxyle ou un radical alkyle en C₁₋₄; R₁₁, R₁₂, R₁₃ et R₁₄, identiques ou différents, représentent un atome d'hydrogène ou un radical alkyle en C₁₋₄ ou hydroxyalkyle en C₁₋₄.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique ou des acides sulfoniques.

Le procédé de teinture selon l'invention consiste, de préférence, à appliquer un mélange, réalisé extemporanément au moment de l'emploi à partir des compositions (A) et (B) décrites ci-dessus, sur les fibres kératiniques sèches ou humides, et à le laisser agir pendant un temps de pause variant, de préférence, de 1 à 60 minutes environ, et plus particulièrement de 10 à 45 minutes environ, à rincer les fibres, puis éventuellement à les laver au shampooing, puis à les rincer à nouveau et à les sécher.

Il est bien entendu que la description qui précède n'a été donnée qu'à titre purement illustratif et non limitatif et que des variantes ou des modifications peuvent y être apportées dans le cadre de la présente invention.

Des exemples concrets illustrant l'invention vont maintenant être donnés sans pour autant présenter un caractère limitatif.

### Exemple

On prépare la composition de teinture d'oxydation suivante :

| | |
|---|---|
| alcool décylique oxyéthyléné (3) | 9 % |
| alcool oléique | 6 % |
| acide oléique | 3 % |
| alkylpolyglycoside(1.4) | 6,9 % |
| alcool éthylique | 6,5 % |
| éther monobutylique d'éthylèneglycol | 10 % |
| laurylhydroxyéthylcellulose quaternisée (commercialisée sous la dénomination Quatrisoft LM 200 par la société Amerchol) | 0,2 % |
| agent séquestrant | qs |
| réducteur | qs |
| parfum | qs |
| agent antioxydant | qs |
| ammoniaque à 20 % | 10 % |
| 1,3-dihydroxybenzène | 0,4 % |
| 3-aminophénol | 0,074 % |
| dichlorhydrate de 1-(β-hydroxyéthoxy)-2,4-diaminobenzène | 0,0094 % |
| 1,3 -dihydroxy-2-méthylbenzène tétrachlorhydrate de N,N'-bis(β-hydroxyéthyl)- | 0,15 % |
| N,N'-bis(4-aminophényl)-1,3-diamino-2-propanol | 0,1 % |
| *para*-phénylènediamine | 0,63 % |
| eau déminéralisée | qs 100 % |

Au moment de l'emploi, on mélange poids pour poids la composition tinctoriale ci-dessus avec une solution de peroxyde d'hydrogène à 20 volumes (6 % en poids).

Le mélange obtenu est appliqué sur des mèches de cheveux gris naturels à 90 % de blancs pendant 30 minutes. Les mèches sont ensuite rincées, lavées avec un shampooing standard, rincées à nouveau puis séchées.

On obtient une nuance châtain clair.

## Revendications

1. Procédé de teinture des fibres kératiniques et en particulier des fibres kératiniques humaines telles que les cheveux, **caractérisé par le fait qu'**il consiste :
- à appliquer sur les fibres une composition de teinture d'oxydation (A) contenant, dans un milieu approprié pour la teinture, au moins un précurseur de colorant d'oxydation et, éventuellement, un ou plusieurs coupleurs, et
- à révéler la couleur en milieu alcalin, neutre ou acide à l'aide d'une composition oxydante (B) contenant un agent oxydant,
l'une au moins des compositions (A) et (B) contenant, en outre, une quantité efficace d'au moins un polymère amphiphile cationique choisi parmi
- les celluloses quaternisées modifiées par des groupements comportant au moins une chaîne grasse, choisis parmi les groupes alkyle, arylalkyle, alkylaryle comportant au moins 8 atomes de carbone, ou des mélanges de ceux-ci, et
- les hydroxyéthylcelluloses quaternisées modifiées par des groupements comportant au moins une chaîne grasse, choisis parmi les groupes alkyle, arylalkyle, alkylaryle comportant au moins 8 atomes de carbone, ou des mélanges de ceux-ci,
les compositions (A) et (B) étant mélangées immédiatement avant l'emploi ou appliquées l'une après l'autre sur les fibres kératiniques.

2. Composition de teinture d'oxydation (A) pour fibres kératiniques, en particulier pour fibres kératiniques humaines telles que les cheveux, **caractérisée par le fait qu'**elle comprend, dans un milieu approprié pour la teinture, au moins un précurseur de colorant d'oxydation, éventuellement un ou plusieurs coupleurs et au moins un polymère amphiphile cationique choisi parmi
- les celluloses quaternisées modifiées par des groupements comportant au moins une chaîne grasse, choisis parmi les groupes alkyle, arylalkyle, alkylaryle comportant au moins 8 atomes de carbone, ou des mélanges de ceux-ci, et
- les hydroxyéthylcelluloses quaternisées modifiées par des groupements comportant au moins une chaîne grasse, choisis parmi les groupes alkyle, arylalkyle, alkylaryle comportant au moins 8 atomes de carbone, ou des mélanges de ceux-ci.

3. Composition oxydante (B) pour teinture d'oxydation des fibres kératiniques et en particulier des fibres kératiniques humaines telles que les cheveux, **caractérisée par le fait qu'**elle comprend au moins un agent oxydant et au moins un polymère amphiphile cationique choisi parmi :
- les celluloses quaternisées modifiées par des groupements comportant au moins une chaîne grasse, choisis parmi les groupes alkyle, arylalkyle, alkylaryle comportant au moins 8 atomes de carbone, ou des mélanges de ceux-ci, et
- les hydroxyéthylcelluloses quaternisées modifiées par des groupements comportant au moins une chaîne grasse, choisis parmi les groupes alkyle, arylalkyle, alkylaryle comportant au moins 8 atomes de carbone, ou des mélanges de ceux-ci.

4. Composition selon la revendication 2 ou 3, **caractérisée par le fait que** les groupes alkyle des celluloses ou hydroxyéthylcelluloses quatemisées sont des groupes alkyle comportant de 8 à 30 atomes de carbone.

5. Composition selon l'une quelconque des revendications 2 à 4, **caractérisée par le fait que** le polymère amphiphile cationique est une hydroxyéthylcellulose quaternisée modifiée par un groupe alkyle en C₁₂ ou C₁₈.

6. Composition selon l'une quelconque des revendications 2 et 4 à 5, **caractérisée par le fait que** les précurseurs de colorants d'oxydation sont choisis parmi les *ortho-* ou *para*-phénylènediamines, les bis-phénylalkylènediamines, les *ortho-* ou *para*-aminophénols, et les bases hétérocycliques ainsi que les sels d'addition d'un acide de ces composés.

7. Composition selon la revendication 6, **caractérisée par le fait que** les précurseurs de colorants d'oxydation sont présents à raison de 0,0005 à 12 % en poids par rapport au poids total de la composition.

8. Composition selon l'une quelconque des revendications 2 et 4 à 7, **caractérisée par le fait que** les coupleurs sont choisis parmi les *méta*-phénylènediamines, les *méta*-aminophénols, les *méta*-diphénols, les coupleurs hétérocycliques et les sels d'addition d'un acide de ces composés.

9. Composition selon la revendication 8, **caractérisée par le fait que** les coupleurs sont présents à raison de 0,0001 à 10 % en poids par rapport au poids total de la composition.

10. Composition selon les revendications 6 et 8, **caractérisée par le fait que** les sels d'addition d'un acide des précurseurs de colorants d'oxydation et des coupleurs sont choisis parmi les chlorhydrates, les bromhydrates, les sulfates, les tartrates, les lactates et les acétates.

11. Composition selon l'une quelconque des revendications 2 et 4 à 10, **caractérisée par le fait qu'**elle contient en outre des colorants directs.

12. Composition selon l'une quelconque des revendications 2 à 11, **caractérisée par le fait qu'**elle contient en outre au moins un polymère substantif cationique ou amphotère.

13. Composition selon la revendication 12, **caractérisée par le fait que** le polymère substantif est un polymère poly(ammonium quaternaire) constitué de motifs récurrents répondant à la formule (IV) suivante :

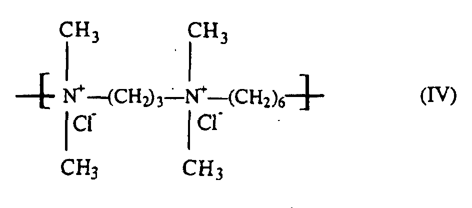

14. Composition selon la revendication 12, **caractérisée par** lefait que le polymère substantif est un polymère poly(ammonium quaternaire) constitué de motifs récurrents répondant à la formule (V) suivante :

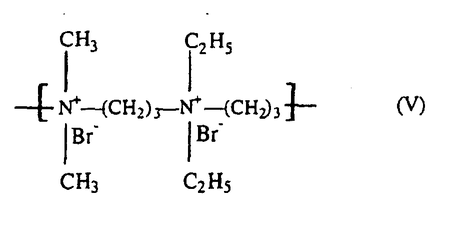

15. Composition selon l'une quelconque des revendications 2 et 4 à 14, **caractérisée par le fait qu'**elle contient, en outre, au moins un agent anti-oxydant, présent en des quantités allant de 0,05 à 3 % en poids par rapport au poids total de la composition.

16. Composition selon l'une quelconque des revendications 2 à 15, **caractérisée par le fait qu'**elle contient, en outre, un ou plusieurs adjuvants choisis parmi les agents séquestrants, les agents de conditionnement du cheveu, notamment des silicones, les agents conservateurs, les agents opacifiants et les agents tensio-actifs anioniques, non-ioniques, amphotères ou leurs mélanges.

17. Composition selon l'une quelconque des revendications 2 et 4 à 16, prête à l'emploi, **caractérisée par le fait qu'**elle contient, en outre, un agent oxydant.

18. Composition selon la revendication 17, **caractérisée par le fait qu'**elle possède un pH allant de 4 à 11.

19. Composition selon la revendication 3 ou 17, **caractérisée par le fait que** l'agent oxydant est choisi parmi le peroxyde d'hydrogène, le peroxyde d'urée, les bromates et les ferricyanures de métaux alcalins, et les sels de peracides.

20. Composition selon la revendication 19, **caractérisée par le fait que** l'agent oxydant est une solution d'eau oxygénée dont le titre varie entre 2,5 et 40 volumes.

21. Composition selon l'une quelconque des revendications 2 à 16, **caractérisée par le fait que** les polymères amphiphiles cationiques sont utilisés en une quantité allant de 0,05 à 10 % en poids, de préférence à raison de 0,1 à 5 % en poids, par rapport au poids de la composition de teinture d'oxydation (A) ou de la composition oxydante (B).

22. Dispositif à plusieurs compartiments, ou "kit", pour la teinture des fibres kératiniques et en particulier des fibres kératiniques humaines telles que les cheveux, **caractérisé par le fait qu'**il comporte au moins deux compartiments dont l'un contient une composition (A) comprenant, dans un milieu approprié pour la teinture, au moins un précurseur de colorant d'oxydation et éventuellement un ou plusieurs coupleurs, et un autre compartiment contient une composition oxydante (B) comprenant au moins un agent oxydant, l'une au moins des compositions (A) et (B) contenant, en outre, une quantité efficace d'au moins un polymère amphiphile cationique choisi parmi :
- les celluloses quaternisées modifiées par des groupements comportant au moins une chaîne grasse, choisis parmi les groupes alkyle, arylalkyle, alkylaryle comportant au moins 8 atomes de carbone, ou des mélanges de ceux-ci, et
- les hydroxyéthylcelluloses quaternisées modifiées par des groupements comportant au moins une chaîne grasse, choisis parmi les groupes alkyle, arylalkyle, alkylaryle comportant au moins 8 atomes de carbone, ou des mélanges de ceux-ci.

## Patentansprüche

1. Verfahren zum Färben von Keratinfasern und insbesondere menschlichen Keratinfasern, wie dem Haar, **dadurch gekennzeichnet, daß** es darin besteht:
- auf die Fasern eine Zusammensetzung zum oxidativen Färben (A) aufzutragen, die in einem zum Färben geeigneten Medium mindestens ein Farbstoffvorprodukt eines Oxidationsfarbstoffes und gegebenenfalls einen oder mehrer Kuppler enthält, und
- die Farbe in einem alkalischen, neutralen oder sauren Medium mit einer oxidierenden Zusammensetzung (B) zu entwickeln, die ein Oxidationsmittel enthält,
wobei mindestens eine der Zusammensetzungen (A) und (B) ferner in einer wirksamen Menge mindestens ein amphiphiles kationisches Polymer enthält, das unter
- quaternisierten Cellulosen, die mit Gruppen mit mindestens einer Fettkette, die unter den Gruppen Alkyl, Arylalkyl oder Alkylaryl mit mindestens 8 Kohlenstoffatomen ausgewählt sind, oder deren Gemischen modifiziert sind, und
- quaternisierten Hydroxyethylcellulosen, die mit Gruppen mit mindestens einer Fettkette, die unter den Gruppen Alkyl, Arylalkyl oder Alkylaryl mit mindestens 8 Kohlenstoffatomen ausgewählt sind, oder deren Gemischen modifiziert sind, ausgewählt ist, wobei die Zusammensetzungen (A) und (B) unmittelbar vor der Anwendung vermischt werden oder nacheinander auf die Keratinfasern aufgebracht werden.

2. Zusammensetzung (A) zum oxidativen Färben von Keratinfasern und insbesondere menschlichen Keratinfasern, wie dem Haar, **dadurch gekennzeichnet, daß** sie in einem zum Färben geeigneten Medium mindestens ein Farbstoffvorprodukt eines Oxidationsfarbstoffes und gegebenenfalls einen oder mehrere Kuppler und mindestens ein amphiphiles kationisches Polymer enthält, das ausgewählt ist unter:
- quaternisierten Cellulosen, die mit Gruppen mit mindestens einer Fettkette, die unter den Gruppen Alkyl, Arylalkyl oder Alkylaryl mit mindestens 8 Kohlenstoffatomen ausgewählt sind, oder deren Gemischen modifiziert sind; und
- quaternisierten Hydroxyethylcellulosen, die mit Gruppen mit mindestens einer Fettkette, die unter den Gruppen Alkyl, Arylalkyl oder Alkylaryl mit mindestens 8 Kohlenstoffatomen ausgewählt sind, oder deren Gemischen modifiziert sind.

3. Oxidierende Zusammensetzung (B) zum oxidativen Färben von Keratinfasern und insbesondere menschlichen Keratinfasern, wie dem Haar, **dadurch gekennzeichnet, daß** sie mindestens ein Oxidationsmittel und mindestens ein amphiphiles kationisches Polymer enthält, das ausgewählt ist unter:
- quaternisierten Cellulosen, die mit Gruppen mit mindestens einer Fettkette, die unter den Gruppen Alkyl, Arylalkyl oder Alkylaryl mit mindestens 8 Kohlenstoffatomen ausgewählt sind, oder deren Gemischen modifiziert sind; und
- quaternisierten Hydroxyethylcellulosen, die mit Gruppen mit mindestens einer Fettkette, die unter den Gruppen Alkyl, Arylalkyl oder Alkylaryl mit mindestens 8 Kohlenstoffatomen ausgewählt sind, oder deren Gemischen modifiziert sind.

4. Zusammensetzung nach Anspruch 2 oder 3, **dadurch gekennzeichnet, daß** die Alkylgruppen der quaternisierten Cellulosen oder Hydroxyethylcellulosen Alkylgruppen mit 8 bis 30 Kohlenstoffatomen sind.

5. Zusammensetzung nach einem der Ansprüche 2 bis 4, **dadurch gekennzeichnet, daß** das kationische amphiphile Polymer eine quaternisierte Hydroxyethylcellulose ist, die mit einer C₁₂-Alkylgruppe oder einer C₁₈-Alkylgruppe modifiziert wurde.

6. Zusammensetzung nach einem der Ansprüche 2 und 4 bis 5, **dadurch gekennzeichnet, daß** die Farbstoffvorprodukte von Oxidationsfarbstoffen unter den o- oder p-Phenylendiaminen, Bisphenylalkylendiaminen, o- oder p-Aminophenolen und heterocyclischen Basen sowie den Additionssalzen dieser Verbindungen mit einer Säure ausgewählt sind.

7. Zusammensetzung nach Anspruch 6, **dadurch gekennzeichnet, daß** die Farbstoffvorprodukte von Oxidationsfarbstoffen in einem Mengenanteil von 0,0005 bis 12 Gew.-%, bezogen auf das Gesamtgewicht der Zusammensetzung, vorliegen.

8. Zusammensetzung nach einem der Ansprüche 2 und 4 bis 7, **dadurch gekennzeichnet, daß** die Kuppler unter den m-Phenylendiaminen, m-Aminophenolen, m-Dihydroxybenzolen und heterocyclischen Kupplern und den Additionssalzen dieser Verbindungen mit einer Säure ausgewählt sind.

9. Zusammensetzung nach Anspruch 8, **dadurch gekennzeichnet, daß** die Kuppler in einem Mengenanteil von 0,0001 bis 10 Gew.-%, bezogen auf das Gesamtgewicht der Zusammensetzung, vorliegen.

10. Zusammensetzung nach den Ansprüchen 6 und 8, **dadurch gekennzeichnet, daß** die Additionssalze der Farbstoffvorprodukte von Oxidationsfarbstoffen und der Kuppler mit einer Säure unter den Hydrochloriden, Hydrobromiden, Sulfaten, Tartraten, Lactaten und Acetaten ausgewählt sind.

11. Zusammensetzung nach einem der Ansprüche 2 und 4 bis 10, **dadurch gekennzeichnet, daß** sie ferner Direktfarbstoffe enthält.

12. Zusammensetzung nach einem der Ansprüche 2 bis 11, **dadurch gekennzeichnet, daß** sie ferner mindestens ein kationisches oder amphoteres substantives Polymer enthält.

13. Zusammensetzung nach Anspruch 12, **dadurch gekennzeichnet, daß** das substantive Polymer ein Poly(quartäres Ammonium)-Polymer mit wiederkehrenden Einheiten der folgenden Formel (IV) ist:

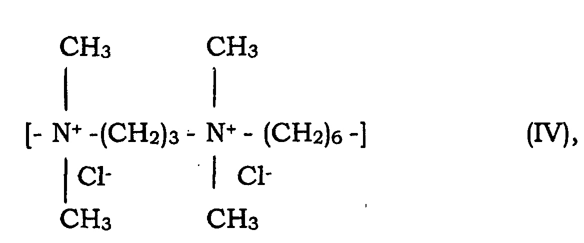

14. Zusammensetzung nach Anspruch 12, **dadurch gekennzeichnet, daß** das substantive Polymer ein Poly(quartäres Ammonium)-Polymer mit wiederkehrenden Einheiten der folgenden Formel (V) ist:

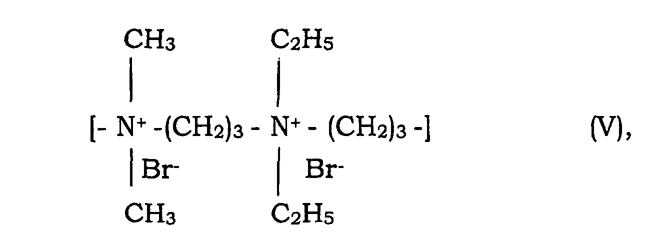

15. Zusammensetzung nach einem der Ansprüche 2 und 4 bis 14, **dadurch gekennzeichnet, daß** sie ferner mindestens ein Antioxidationsmittel enthält, das in einer Menge von 0,05 bis 3 Gew.-%, bezogen auf das Gesamtgewicht der Zusammensetzung, vorliegt.

16. Zusammensetzung nach einem der Ansprüche 2 bis 15, **dadurch gekennzeichnet, daß** sie ferner einen oder mehrere Zusatzstoffe enthält, die unter den Maskierungsmitteln, Konditioniermitteln für das Haar und insbesondere Siliconen, Konservierungsmitteln, Trübungsmitteln und anionischen, nichtionischen oder amphoteren grenzflächenaktiven Stoffen oder deren Gemischen ausgewählt sind.

17. Zusammensetzung nach einem der Ansprüche 2 und 4 bis 16, die gebrauchsfertig ist, **dadurch gekennzeichnet, daß** sie ferner ein Oxidationsmittel enthält.

18. Zusammensetzung nach Anspruch 17, **dadurch gekennzeichnet, daß** ihr pH-Wert im Bereich von 4 bis 11 liegt.

19. Zusammensetzung nach Anspruch 3 oder 17, **dadurch gekennzeichnet, daß** das Oxidationsmittel unter Wasserstoffperoxid, Harnstoffperoxid, Bromaten und Ferricyaniden von Alkalimetallen und Salzen von Persäuren ausgewählt ist.

20. Zusammensetzung nach Anspruch 19, **dadurch gekennzeichnet, daß** das Oxidationsmittel eine Wasserstoffperoxidlösung mit einem Titer von 2,5 bis 40 Volumina ist.

21. Zusammensetzung nach einem der Ansprüche 2 bis 16, **dadurch gekennzeichnet, daß** die amphiphilen kationischen Polymere in einem Mengenanteil von 0,05 bis 10 Gew.-% und vorzugsweise 0,1 bis 5 Gew.-%, bezogen auf das Gesamtgewicht der Zusammensetzung zum oxidativen Färben (A) oder der oxidierenden Zusammensetzung (B), verwendet werden.

22. Vorrichtung mit mehreren Abteilungen oder "Kit" zum Färben von Keratinfasern und insbesondere menschlichen Keratinfasern, wie dem Haar, **dadurch gekennzeichnet, daß** sie mindestens zwei Abteilungen aufweist, wobei eine Abteilung eine Zusammensetzung (A), die in einem zum Färben geeigneten Medium mindestens ein Farbstoffvorprodukt eines Oxidationsfarbstoffes und gegebenenfalls einen oder mehrere Kuppler enthält, und eine weitere Abteilung die oxidierende Zusammensetzung (B) enthält, die mindestens ein Oxidationsmittel enthält, wobei mindestens eine der Zusammensetzungen (A) und (B) ferner in einer wirksamen Menge mindestens ein amphiphiles kationisches Polymer enthält, das ausgewählt ist unter
- quaternisierten Cellulosen, die mit Gruppen mit mindestens einer Fettkette, die unter den Gruppen Alkyl, Arylalkyl oder Alkylaryl mit mindestens 8 Kohlenstoffatomen ausgewählt sind, oder deren Gemischen modifiziert sind; und
- quaternisierten Hydroxyethylcellulosen, die mit Gruppen mit mindestens einer Fettkette, die unter den Gruppen Alkyl, Arylalkyl oder Alkylaryl mit mindestens 8 Kohlenstoffatomen ausgewählt sind, oder deren Gemischen modifiziert sind.

## Claims

1. Process for dyeing keratin fibres, and in particular human keratin fibres such as the hair, which consists in:
- applying to the fibres an oxidation dye composition (A) containing, in a medium which is suitable for dyeing, at least one oxidation dye precursor and optionally one or more couplers, and
- in developing the colour in alkaline, neutral or acidic medium using an oxidizing composition (B) containing an oxidizing agent,
at least one of the compositions (A) and (B) also containing an effective amount of at least one cationic amphiphilic polymer chosen from:
- quaternized celluloses modified with groups containing at least one fatty chain, chosen from alkyl, arylalkyl and alkylaryl groups containing at least 8 carbon atoms, or mixtures thereof, and
- quaternized hydroxyethylcelluloses modified with groups containing at least one fatty chain, chosen from alkyl, arylalkyl and alkylaryl groups containing at least 8 carbon atoms, or mixtures thereof,
the compositions (A) and (B) being mixed together immediately before use or applied one after the other to the keratin fibres.

2. Oxidation dye composition (A) for keratin fibres, in particular for human keratin fibres such as the hair, **characterized in that** it comprises, in a medium which is suitable for dyeing, at least one oxidation dye precursor, optionally one or more couplers and at least one cationic amphiphilic polymer chosen from:
- quaternized celluloses modified with groups containing at least one fatty chain, chosen from alkyl, arylalkyl and alkylaryl groups containing at least 8 carbon atoms, or mixtures thereof, and
- quaternized hydroxyethylcelluloses modified with groups containing at least one fatty chain, chosen from alkyl, arylalkyl and alkylaryl groups containing at least 8 carbon atoms, or mixtures thereof.

3. Oxidizing composition (B) for the oxidation dyeing of keratin fibres, and in particular human keratin fibres such as the hair, **characterized in that** it comprises at least one oxidizing agent and at least one cationic amphiphilic polymer chosen from:
- quaternized celluloses modified with groups containing at least one fatty chain, chosen from alkyl, arylalkyl and alkylaryl groups containing at least 8 carbon atoms, or mixtures thereof, and
- quaternized hydroxyethylcelluloses modified with groups containing at least one fatty chain, chosen from alkyl, arylalkyl and alkylaryl groups containing at least 8 carbon atoms, or mixtures thereof.

4. Composition according to Claim 2 or 3, **characterized in that** the alkyl groups of the quaternized celluloses or hydroxyethylcelluloses are alkyl groups containing from 8 to 30 carbon atoms.

5. Composition according to any one of Claims 2 to 4, **characterized in that** the cationic amphiphilic polymer is a quaternized hydroxyethylcellulose modified with a C₁₂ or C₁₈ alkyl group.

6. Composition according to any one of Claims 2, 4 and 5, **characterized in that** the oxidation dye precursors are chosen from ortho- or para-phenylenediamines, bis(phenyl)alkylenediamines, ortho- or paraaminophenols and heterocyclic bases, as well as the addition salts of these compounds with an acid.

7. Composition according to Claim 6, **characterized in that** the oxidation dye precursors are present in a proportion of from 0.0005 to 12% by weight relative to the total weight of the composition.

8. Composition according to any one of Claims 2 and 4 to 7, **characterized in that** the couplers are chosen from meta-phenylenediamines, meta-aminophenols, meta-diphenols and heterocyclic couplers, and the addition salts of these compounds with an acid.

9. Composition according to Claim 8, **characterized in that** the couplers are present in a proportion of from 0.0001 to 10% by weight relative to the total weight of the composition.

10. Composition according to Claims 6 and 8,
**characterized in that** the addition salts with an acid of the oxidation dye precursors and of the couplers are chosen from the hydrochlorides, hydrobromides, sulphates, tartrates, lactates and acetates.

11. Composition according to any one of Claims 2 and 4 to 10, **characterized in that** it also contains direct dyes.

12. Composition according to any one of Claims 2 to 11, **characterized in that** it also contains at least one cationic or amphoteric substantive polymer.

13. Composition according to Claim 12, **characterized in that** the substantive polymer is a poly(quaternary ammonium) polymer consisting of repeating units corresponding to formula (IV) below:

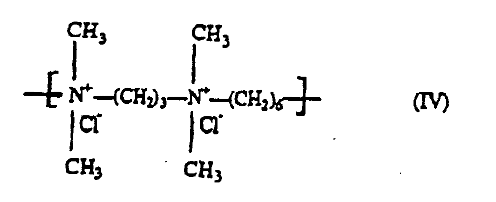

14. Composition according to Claim 12, **characterized in that** the substantive polymer is a poly(quaternary ammonium) polymer consisting of repeating units corresponding to formula (V) below:

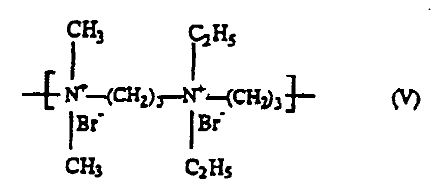

15. Composition according to any one of Claims 2 and 4 to 14, **characterized in that** it also contains at least one antioxidant, present in amounts ranging from 0.05 to 3% by weight relative to the total weight of the composition.

16. Composition according to any one of Claims 2 to 15, **characterized in that** it also contains one or more adjuvants chosen from sequestering agents, hair conditioners, in particular silicones, preserving agents, opacifiers and anionic, nonionic or amphoteric surfactants, or mixtures thereof.

17. Ready-to-use composition according to any one of Claims 2 and 4 to 16, **characterized in that** it also contains an oxidizing agent.

18. Composition according to Claim 17, **characterized in that** it has a pH ranging from 4 to 11.

19. Composition according to Claim 3 or 17, **characterized in that** the oxidizing agent is chosen from hydrogen peroxide, urea peroxide, alkali,metal bromates and ferricyanides, and salts of peracids.

20. Composition according to Claim 19, **characterized in that** the oxidizing agent is an aqueous hydrogen peroxide solution whose titre ranges between 2.5 and 40 volumes.

21. Composition according to any one of Claims 2 to 16, **characterized in that** the cationic amphiphilic polymers are used in an amount ranging from 0.05 to 10% by weight, preferably in a proportion of from 0.1 to 5% by weight, relative to the weight of the oxidation dye composition (A) or the oxidizing composition (B).

22. Multi-compartment device or "kit" for dyeing keratin fibres, and in particular human keratin fibres such as the hair, **characterized in that** it contains at least two compartments, one of which contains a composition (A) comprising, in a medium which is suitable for dyeing, at least one oxidation dye : precursor and optionally one or more couplers, and another compartment contains an oxidizing composition (B) comprising at least one oxidizing agent, at least one of the compositions (A) and (B) also containing an effective amount of at least one cationic amphiphilic polymer chosen from:
- quaternized celluloses modified with groups containing at least one fatty chain, chosen from alkyl, arylalkyl and alkylaryl groups containing at least 8 carbon atoms, or mixtures thereof, and
- quaternized hydroxyethylcelluloses modified with groups containing at least one fatty chain, chosen from alkyl, arylalkyl and alkylaryl groups containing at least 8 carbon atoms, or mixtures thereof.
